# EUROPEAN PATENT APPLICATION

(11) **EP 4 506 329 A1**
(43) Date of publication of application: **12.02.2025**
(21) Application number: 23780174.1
(22) Date of filing: 24.03.2023
(51) Int. Cl.: C07C 51/09, C07C 59/52, C07D 211/22, C07D 405/14

(54) **METHOD FOR PRODUCING ARYL ESTER-CONTAINING CARBOXYLIC ACID AND METHOD FOR PRODUCING CATIONIC LIPID**

(30) Priority: 28.03.2022 JP 2022051920
(71) Applicant: NOF Corporation, Shibuya-ku Tokyo 150-6019 (JP)
(72) Inventor: MOTOUSU, Ryosuke, Kawasaki-shi, Kanagawa 210-0865 (JP); OTA, Masaki, Kawasaki-shi, Kanagawa 210-0865 (JP); OZONE, Daiki, Kawasaki-shi, Kanagawa 210-0865 (JP); SATO, Masayuki, Kawasaki-shi, Kanagawa 210-0865 (JP)
(74) Representative: Weickmann & Weickmann PartmbB
(86) International application number: PCT/JP2023/011837
(87) International publication number: WO 2023/190164

(57) **Abstract**

The present invention provides a method for producing a high-purity carboxylic acid containing aryl ester that suppresses generation of oligomers and does not require a purification step with low productivity such as column chromatography and the like, and a method for producing cationic lipids. A method for producing an aryl ester-containing carboxylic acid (1), including the following steps: 1) a step of reacting a compound (7) using potassium tert-butoxide and tert-butyl alcohol in the presence of an aprotic solvent to obtain a compound (11), 2) a step of reacting the compound (11) with a compound (13) using a condensing agent to obtain a compound (12), and 3) a step of deprotecting the tert-butyl group of the compound (12) by using trifluoromethanesulfonic acid, methanesulfonic acid, a combination of TMS-Cl and sodium iodide, or TMS-I in an organic solvent to obtain the compound (1) (A is phenyl having -O-C(=O)R²⁰ group, etc., and B is phenyl having hydroxy group, etc.).

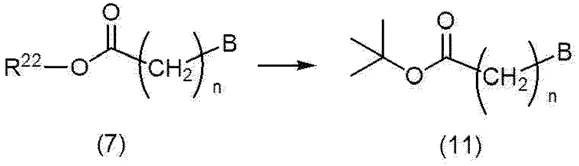

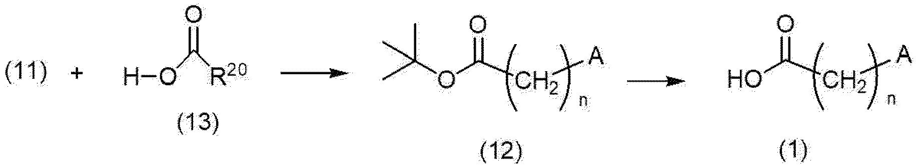

## Description

### [Technical Field]

The present invention relates to a method for producing carboxylic acids containing aryl esters and a method for producing cationic lipids.

### [Background Art]

Carboxylic acids containing aryl esters currently play an important role as partial structures of various acetic acid derivative pharmaceuticals, as represented by non-steroidal antiinflammatory agents of aryl acetic acid derivatives (see Non Patent Literature 1). Furthermore, they also play an important role as partial structures of LNP raw materials, as shown by the contribution of high degradability of aryl esters to the sustained release of lipid nanoparticles (LNPs) in mRNA therapeutic drug use in Non Patent Literature 2.

On the other hand, in order to produce the above-mentioned compound containing an aryl ester, a carboxylic acid containing an aryl ester is used. When an aryl ester is to be formed using a carboxylic acid having a phenolic hydroxyl group, a method using an active acylating agent such as acid chloride, acid anhydride, and the like as described in Patent Literature 1 and the like or a method using a condensing agent such as DCC, polyphosphoric acid, and the like is considered to be desirable. However, it is known that in the above-mentioned method using a condensing agent, the condensing agent also acts on the carboxyl group of the compound containing a phenolic hydroxyl group to produce an oligomer.

It is also known that even when a carboxylic acid is converted into an active acyl group by a method using an acid anhydride or an acid chloride in advance to give selectivity to the carbonyl group and then a phenolic hydroxyl group is reacted, the carboxyl group contained in the phenolic hydroxyl group acts on the active acyl group and not only the desired aryl ester but also a hetero acid anhydride in which the carboxyl group acts is formed (Non Patent Literature 3). In this hetero acid anhydride, the action of the phenolic hydroxyl group produces an oligomer similar to that in the case of using a condensing agent.

### [Citation List]

### [Patent Literature]

[Patent Literature 1]
   JP 2011-105667 A
[Patent Literature 2]
   WO 2019/188867
[Patent Literature 3]
   WO 2021/195529

### [Non Patent Literature]

[Non Patent Literature 1]
   The Journal of the Japanese Society of Internal Medicine 100:2888-2901, 2011
[Non Patent Literature 2]
   Adv. Funct. Mater. 30, 1910575 (2020)
[Non Patent Literature 3]
   Chem. Papers, 1997, 51, 111-116

### [Summary of Invention]

### [Technical Problem]

However, since the above-mentioned oligomers have reactivity and properties similar to those of monomeric compounds, they may sometimes cause various side reactions or may be difficult to remove during purification.

As one embodiment, when oleic anhydride is reacted with 4-hydroxyphenylacetic acid during the synthesis of 4-oleoyloxyphenylacetic acid described in the examples of Patent Literature 2, oligomers of 4-hydroxyphenylacetic acid are produced, and therefore, six times of extraction using acetonitrile and purification using silica gel column chromatography are required, and the amount of 4-oleoyloxyphenylacetic acid obtained is small relative to the amount of oleic anhydride used. Therefore, this is not a satisfactory level for general production methods.

Furthermore, since the pKa of the phenolic hydroxyl group is 8 to 11, which is higher in acidity compared with alcohols and water, aryl esters are generally known to be highly decomposable in water or alcohol, and are particularly known to be easily decomposed under basic conditions, thus posing a problem that cannot be ignored when producing a desired carboxylic acid containing aryl ester.

In view of the above-mentioned problems, the present invention aims to provide a method for producing a high-purity carboxylic acid containing aryl ester and a method for producing a cationic lipid, which suppress the production of oligomers and do not require a purification step with low productivity such as column chromatography and the like.

### [Solution to Problem]

The inventors have conducted intensive studies and found that high-purity carboxylic acids containing aryl ester can be produced while essentially avoiding by-production of oligomers and without performing purification methods with low productivity represented by column chromatography, by transesterifying, using potassium tert-butoxide, a hydroxyarylcarboxylic acid esterified with a methyl group or an ethyl group to introduce a tert-butyl group only into the carboxylic acid and, after esterification of the hydroxy group, deprotecting the tert-butyl group with a specific deprotecting agent, which resulted in the completion of the present invention.

That is, the present invention encompasses the following:
[1] A method for producing an aryl ester-containing carboxylic acid represented by the following formula (1), comprising the following three steps:
   (step 1) a step of reacting a compound represented by the following formula (7) using potassium tert-butoxide and tert-butyl alcohol in the presence of an aprotic solvent to obtain a compound represented by the following formula (11),
   (step 2) a step of reacting the compound represented by the following formula (11) with a compound represented by the following formula (13) using a condensing agent to obtain a compound represented by the following formula (12), and
   (step 3) a step of deprotecting the tert-butyl group of the compound represented by the following formula (12) by using trifluoromethanesulfonic acid, methanesulfonic acid, a combination of trimethylsilyl chloride (TMS-Cl) and sodium iodide, or trimethylsilyl iodide (TMS-I) in an organic solvent to obtain a compound represented by the following formula (1); 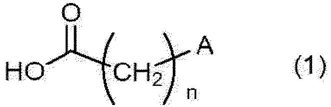
   in the formula (1), A is a group represented by the following formula (2), (3), or (4), and n is an integer of 1 to 10 indicating the number of repeating units of methylene group,

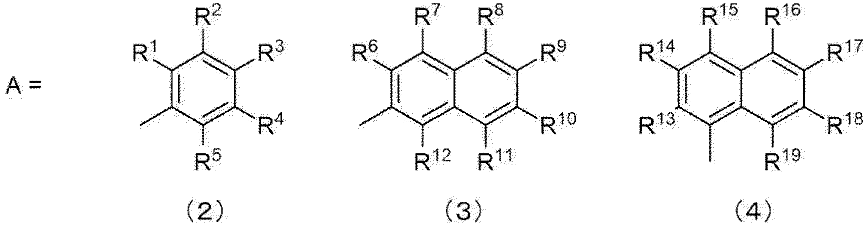

   in the formula (2), at least one selected from R¹ to R⁵ is a group represented by the following formula (5), and the rest are each independently a hydrogen atom, a halogen atom, or an alkyl group,
   in the formula (3), at least one selected from R⁶ to R¹² is a group represented by the following formula (5), and the rest are each independently a hydrogen atom, a halogen atom, or an alkyl group,
   in the formula (4), at least one selected from R¹³ to R¹⁹ is a group represented by the following formula (5), and the rest are each independently a hydrogen atom, a halogen atom, or an alkyl group, 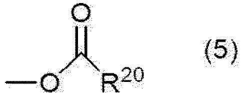
   in the formula (5), R²⁰ is a structure of I, II, or III shown below,
      I: a linear aliphatic hydrocarbon group having 12 to 22 carbon atoms, a branched aliphatic hydrocarbon group having 12 to 37 carbon atoms, a liposoluble vitamin residue, or a sterol derivative residue,
      II: a group represented by the following formula (6) [0019] 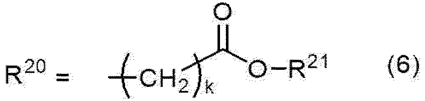 in the formula (6), k is an integer of 2 to 10 indicating the number of repeating units of methylene group, and R²¹ is a linear aliphatic hydrocarbon group having 12 to 22 carbon atoms, a branched aliphatic hydrocarbon group having 12 to 37 carbon atoms, a liposoluble vitamin residue, or a sterol derivative residue,
      III: a group containing a benzene ring and a guanidino group, and consisting of 7 or 8 carbon atoms, 3 nitrogen atoms, and a hydrogen atom; 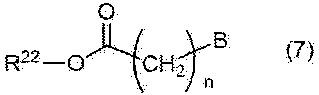
   in the formula (7), B is a group represented by the following formula (8), (9), or (10), n is an integer of 1 to 10 indicating the number of repeating units of methylene group, and R²² is methyl or ethyl, 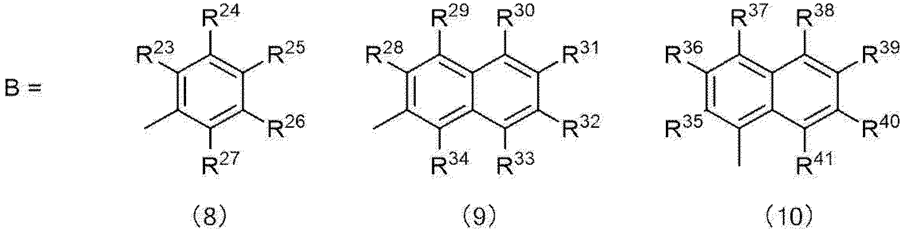
   in the formula (8), at least one selected from R²³ to R²⁷ is a hydroxy group, and the rest are each independently a hydrogen atom, a halogen atom, or an alkyl group,
   in the formula (9), at least one selected from R²¹ to R³⁴ is a hydroxy group, and the rest are each independently a hydrogen atom, a halogen atom, or an alkyl group,
   in the formula (10), at least one selected from R³⁵ to R⁴¹ is a hydroxy group, and the rest are each independently a hydrogen atom, a halogen atom, or an alkyl group; 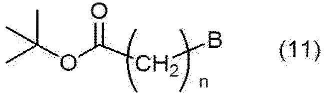
   in the formula (11), B is a group represented by the aforementioned formula (8), (9), or (10), and n is an integer of 1 to 10 indicating the number of repeating units of methylene group; 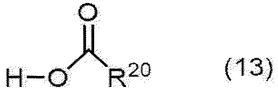
   in the formula (13), R²⁰ is a structure of I, II, or III shown in the above; 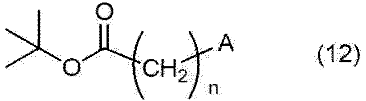
   in the formula (12), A is a group represented by the aforementioned formula (2), (3), or (4), and n is an integer of 1 to 10 indicating the number of repeating units of methylene group.
[2] The production method of [1], wherein A in the aforementioned formulas (1) and (12) is a group represented by the aforementioned formula (2), and B in the aforementioned formulas (7) and (11) is a group represented by the aforementioned formula (8).
[3] The production method of [2], wherein, in the aforementioned formula (2), at least one selected from R¹ to R⁵ is a group represented by the aforementioned formula (5), and the rest are each independently a hydrogen atom, and in the aforementioned formula (8), at least one selected from R²³ to R²⁷ is a hydroxy group, and the rest are each independently a hydrogen atom.
[4] The production method of [2] or [3], wherein, in the aforementioned formula (2), R³ is a group represented by the aforementioned formula (5), and R¹, R², R⁴, and R⁵ are each independently a hydrogen atom, and in the aforementioned formula (8), R²⁵ is a hydroxy group, and R²³, R²⁴, R²⁶, and R²⁷ are each independently a hydrogen atom.
[5] The production method of any one of [1] to [4], wherein R²⁰ in the aforementioned formulas (5) and (13) is a structure of the aforementioned I or II.
[6] The production method of any one of [1] to [5], wherein n is 1.
[7] The production method of any one of [1] to [6], wherein methanesulfonic acid is used in the aforementioned (step 3).
[8] The production method of any one of [1] to [7], wherein the aprotic solvent in the aforementioned (step 1) is toluene.
[9] The production method of any one of [1] to [8], wherein the condensing agent in the aforementioned (step 2) is selected from the group consisting of 1-(3-dimethylaminopropyl)-3-ethylcarbodiimide hydrochloride (EDC hydrochloride), 1-(3-dimethylaminopropyl)-3-ethylcarbodiimide (EDC), N,N'-dicyclohexylcarbodiimide (DCC), and N,N'-diisopropylcarbodiimide (DIC).
[10] The production method of any one of [1] to [9], wherein the organic solvent in the aforementioned (step 3) is acetonitrile, dichloromethane or chloroform, or a mixture thereof.
[11] The production method of any one of [1] to [10], wherein, R²² in the aforementioned formula (7) is methyl.
[12] A method for producing a cationic lipid, comprising the (step 1), (step 2) and (step 3) of any one of [1] to [11], and (step 4) a step of condensing the obtained aryl ester-containing carboxylic acid represented by the formula (1) and a compound having a disulfide bond, at least one tertiary nitrogen, and at least one hydroxy group or amino group.
[13] The production method of [12], wherein the aforementioned compound is a compound represented by the formula (14), and
   in the aforementioned (step 4), the aforementioned aryl ester-containing carboxylic acid and the hydroxy groups of the aforementioned compound are condensed to obtain a cationic lipid represented by the formula (20): 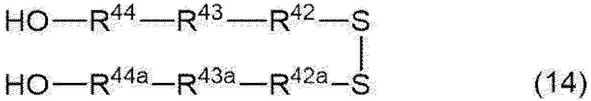 in the formula (14),
   R⁴² and R^{42a} are each independently an alkylene group having 1 to 6 carbon atoms,
   R⁴³ and R^{43a} are each independently a non-cyclic alkyl tertiary amino group having 1 to 6 carbon atoms and one tertiary amino group, or a cyclic alkylene tertiary amino group having 2 to 5 carbon atoms and 1 to 2 tertiary amino groups, and
   R⁴⁴ and R^{44a} are each independently an alkylene group or an oxydialkylene group each having not more than 8 carbon atoms; 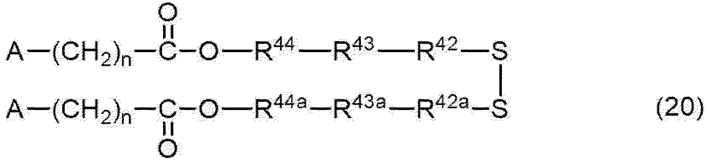 in the formula (20), each symbol is as defined above.

### [Advantageous Effects of Invention]

The production method of the present invention can essentially avoid the by-production of oligomers. Furthermore, it can suppress the decomposition of aryl esters and can provide a high-purity carboxylic acid containing aryl ester without requiring a purification step with low productivity such as column chromatography. Therefore, the method can also be applied as a method for producing raw materials in the pharmaceutical field, where high purity products are particularly required.

Furthermore, in the synthesis of cationic lipids using carboxylic acids containing aryl esters as raw materials as shown in Patent Literature 2, it is possible to synthesize the lipids while essentially avoiding the generation of impurities derived from oligomers.

### [Description of Embodiments]

While the embodiments of the present invention are explained in the following, the present invention is not limited thereto. In the following embodiments, a method for producing a carboxylic acid containing an aryl ester and a method for producing a cationic lipid are described.

### [Production method of aryl ester-containing carboxylic acid]

The present invention provides a production method of a aryl ester-containing carboxylic acid represented by the formula (1), including step 1 to step 3.

In the formulas (1) and (12), A is a group represented by the formula (2), (3), or (4), preferably a group represented by the formula (2).

In the formulas (1) and (12), n is an integer of 1 to 10, preferably an integer of 1 to 5, more preferably 1, which indicates the number of repeating units of methylene group.

In the formula (2), at least one selected from R¹ to R⁵ is a group represented by the formula (5), preferably one selected from R¹ to R⁵ is a group represented by the formula (5).

In R¹ to R⁵ of the formula (2), the rest of the substituents other than the group represented by the formula (5) are each independently a hydrogen atom, a halogen atom, or an alkyl group, preferably a hydrogen atom.

In the formula (3), at least one selected from R⁶ to R¹² is a group represented by the formula (5), preferably one selected from R⁶ to R¹² is a group represented by the formula (5).

In R⁶ to R¹² of the formula (3), the rest of the substituents other than the group represented by the formula (5) are each independently a hydrogen atom, a halogen atom, or an alkyl group, preferably a hydrogen atom.

In the formula (4), at least one selected from R¹³ to R¹⁹ is a group represented by the formula (5), preferably one selected from R¹³ to R¹⁹ is a group represented by the formula (5).

In R¹³ to R¹⁹ of the formula (4), the rest of the substituents other than the group represented by the formula (5) are each independently a hydrogen atom, a halogen atom, or an alkyl group, preferably a hydrogen atom.

In the present invention, the "alkyl group" may be linear or branched. The carbon number of the alkyl group is preferably 1 to 6, more preferably 1 to 4. Specific examples of the alkyl group include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, and the like, preferably methyl group, ethyl group, propyl group, isopropyl group, n-butyl group, sec-butyl group, isobutyl group, and tert-butyl group.

In the formulas (5) and (13), R²⁰ is a structure of I, II, or III shown above, preferably the structure of I or II, most preferably the structure of I.

The structure of I is a linear aliphatic hydrocarbon group having 12 to 22 carbon atoms, a branched aliphatic hydrocarbon group having 12 to 37 carbon atoms, a liposoluble vitamin residue, or a sterol derivative residue. The aliphatic hydrocarbon group may be linear or branched, and it may be saturated or unsaturated.

When R²⁰ in the formulas (5) and (13) is a linear aliphatic hydrocarbon group described in I, the carbon number of the R²⁰ is 12 to 22, preferably 12 to 20, more preferably 14 to 20. Specific examples thereof include dodecyl group, tridecyl group, tetradecyl group, pentadecyl group, hexadecyl group, heptadecyl group, octadecyl group, nonadecyl group, icosyl group, henicosyl group, docosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, heneicosadienyl group, docosadienyl group, octadecatrienyl group, icosatrienyl group, icosatetraenyl group, icosapentaenyl group, docosahexaenyl group, and the like.

When R²⁰ in the formulas (5) and (13) is a branched aliphatic hydrocarbon group described in I, the carbon number of the R²⁰ is 12 to 37, preferably 14 to 37. Specific examples thereof include isostearyl group, 1-hexylheptyl group, 1-hexylnonyl group, 1-octylnonyl group, 1-octylundecyl group, 1-decylundecyl group, 1-dodecyltridecyl group, 1-tetradecylpentadecyl group, 1-hexadecylheptadecyl group, 1-octadecylnonadecyl group, and the like, preferably, 1-hexylheptyl group, 1-hexylnonyl group, 1-octylnonyl group, 1-octylundecyl group, 1-decylundecyl group, 1-dodecyltridecyl group, 1-tetradecylpentadecyl group, and 1-hexadecylheptadecyl group.

When R²⁰ in the formulas (5) and (13) is an unsaturated aliphatic hydrocarbon group described in I, the carbon number of the aliphatic hydrocarbon group is 12 to 22, preferably 12 to 20, more preferably 14 to 20. The aliphatic hydrocarbon group generally contains 1 to 6, preferably 1 to 3, more preferably 1 unsaturated bond. While the unsaturated bond includes a carbon-carbon double bond and a carbon-carbon triple bond, it is preferably a carbon-carbon double bond. Specific examples of the aliphatic hydrocarbon group having unsaturated bond(s) and 12 to 22 carbon atoms include dodecenyl group, tridecenyl group, tetradecenyl group, pentadecenyl group, hexadecenyl group, heptadecenyl group, octadecenyl group, nonadecenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, and the like.

In the present invention, examples of the liposoluble vitamin residue include residues derived from liposoluble vitamins, or residues in which a hydroxyl group, which is a functional group in a liposoluble vitamin, is converted to another reactive functional group, and the like. Examples include monovalent groups having a structure obtained by removing a hydroxyl group from a liposoluble vitamin, monovalent groups having a structure obtained by removing a hydrogen atom from a hydroxyl group of a liposoluble vitamin, and the like. The liposoluble vitamins include retinol, ergosterol, 7-dehydrocholesterol, calciferol, cholecalciferol, dihydroergocalciferol, dihydrotachysterol, tocopherol, tocotrienol, and the like, preferably tocopherol.

In the present invention, examples of the sterol derivative residue include residues derived from sterol derivatives, or residues in which a hydroxyl group, which is a functional group in a sterol derivative, is converted to another reactive functional group, and the like. Examples include monovalent groups having a structure obtained by removing a hydroxyl group from a sterol derivative, monovalent groups having a structure obtained by removing a hydrogen atom from a hydroxyl group of a sterol derivative, and the like. The sterol derivatives include cholesterol, cholestanol, stigmasterol, β-sitosterol, lanosterol, ergosterol, and the like, preferably cholesterol or cholestanol.

When R²⁰ in the formulas (5) and (13) is a liposoluble vitamin residue in I, examples thereof include monovalent groups having a structure obtained by removing a hydroxyl group from a liposoluble vitamin. Specific examples of the liposoluble vitamin include retinol, ergosterol, 7-dehydrocholesterol, calciferol, cholecalciferol, dihydroergocalciferol, dihydrotachysterol, tocopherol, tocotrienol, and the like, preferably tocopherol.

When R²⁰ in the formulas (5) and (13) is a sterol derivative residue in I, examples thereof include monovalent groups having a structure obtained by removing a hydroxyl group from a sterol derivative, and the like. Specific examples of the sterol derivative include cholesterol, cholestanol, stigmasterol, β-sitosterol, lanosterol, ergosterol, and the like, and it is preferably cholesterol or cholestanol.

The structure of II is a group represented by the aforementioned formula (6), and k is an integer of 2 to 10, preferably 2 to 7, indicating the number of repeating units of methylene group.

R²¹ in the formula (6) is a linear aliphatic hydrocarbon group having 12 to 22 carbon atoms, a branched aliphatic hydrocarbon group having 12 to 37 carbon atoms, a liposoluble vitamin residue, or a sterol derivative residue. The aliphatic hydrocarbon group may be linear or branched, and may be saturated or unsaturated.

When R²¹ in the formula (6) is a linear aliphatic hydrocarbon group, the carbon number of the R²¹ is 12 to 22, preferably 12 to 20, more preferably 14 to 20. Specific examples thereof include dodecyl group, tridecyl group, tetradecyl group, pentadecyl group, hexadecyl group, heptadecyl group, octadecyl group, nonadecyl group, icosyl group, henicosyl group, docosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, heneicosadienyl group, docosadienyl group, octadecatrienyl group, icosatrienyl group, icosatetraenyl group, icosapentaenyl group, docosahexaenyl group, and the like.

When R²¹ in the formula (6) is a branched aliphatic hydrocarbon group, the carbon number of the R²¹ is 12 to 37, preferably 14 to 37. Specific examples thereof include isostearyl group, 1-hexylheptyl group, 1-hexylnonyl group, 1-octylnonyl group, 1-octylundecyl group, 1-decylundecyl group, 1-dodecyltridecyl group, 1-tetradecylpentadecyl group, 1-hexadecylheptadecyl group, 1-octadecylnonadecyl group, and the like, particularly preferably 1-hexylheptyl group, 1-hexylnonyl group, 1-octylnonyl group, 1-octylundecyl group, 1-decylundecyl group, 1-dodecyltridecyl group, 1-tetradecylpentadecyl group, and 1-hexadecylheptadecyl group.

When R²¹ in the formula (6) is an unsaturated aliphatic hydrocarbon group, the carbon number of the aliphatic hydrocarbon group is 12 to 22, preferably 12 to 20, more preferably 14 to 20. The number of unsaturated bonds contained in the aliphatic hydrocarbon group is generally 1 to 6, preferably 1 to 3, more preferably 1. The unsaturated bond includes carbon-carbon double bond and carbon-carbon triple bond, preferably carbon-carbon double bond. Specific examples of the aliphatic hydrocarbon group having unsaturated bond(s) and 12 to 22 carbon atoms include dodecenyl group, tridecenyl group, tetradecenyl group, pentadecenyl group, hexadecenyl group, heptadecenyl group, octadecenyl group, nonadecenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, and the like.

When R²¹ in the formula (6) is a liposoluble vitamin residue, examples thereof include monovalent groups having a structure obtained by removing a hydroxyl group from liposoluble vitamin. Specific examples of the liposoluble vitamin include retinol, ergosterol, 7-dehydrocholesterol, calciferol, cholecalciferol, dihydroergocalciferol, dihydrotachysterol, tocopherol, tocotrienol, and the like, preferably tocopherol.

When R²¹ in the formula (6) is a sterol derivative residue, examples thereof include monovalent groups having a structure obtained by removing a hydroxyl group from a sterol derivative and the like. Specific examples of the sterol derivative include cholesterol, cholestanol, stigmasterol, β-sitosterol, lanosterol, and ergosterol, and the like, and it is preferably cholesterol or cholestanol.

The structure of III is a group containing a benzene ring and a guanidino group, and consisting of 7 or 8 carbon atoms, 3 nitrogen atoms, and hydrogen atoms, and is preferably a group containing a benzene ring and a guanidino group, and consisting of 7 carbon atoms, 3 nitrogen atoms, and hydrogen atoms. Here, the number of carbon atoms is the total number of the whole group including the number of carbon atoms in the benzene ring and the guanidino group. The number of nitrogen atoms is the number of nitrogen atoms in the guanidino group. The structure of III is more preferably a phenyl group substituted with a guanidino group.

In the formulas (7) and (11), B is a group represented by the formula (8), (9), or (10), and is preferably a group represented by the formula (8). Furthermore, R²² is methyl or ethyl, and is preferably methyl.

In the formulas (7) and (11), n is an integer of 1 to 10 indicating the number of repeating units of the methylene group, and is preferably an integer of 1 to 5, more preferably 1.

In the formula (8), at least one selected from R²³ to R²⁷ is a hydroxy group, preferably one selected from R²³ to R²⁷ is a hydroxy group.

In R²³ to R²⁷ in the formula (8), the rest of the substituents other than hydroxy group are each independently a hydrogen atom, a halogen atom, or an alkyl group, preferably a hydrogen atom.

In a preferred embodiment, in the formula (8), R²⁵ is a hydroxy group, and R²³, R²⁴, R²⁶, and R²⁷ are each independently a hydrogen atom.

In the formula (9), at least one selected from R²⁸ to R³⁴ is a hydroxy group, preferably one selected from R²⁸ to R³⁴ is a hydroxy group.

In R²⁸ to R³⁴ in the formula (9), the rest of the substituents other than hydroxy group are each independently a hydrogen atom, a halogen atom, or an alkyl group, preferably a hydrogen atom.

In the formula (10), at least one selected from R³⁵ to R⁴¹ is a hydroxy group, preferably one selected from R³⁵ to R⁴¹ is a hydroxy group.

In R³⁵ to R⁴¹ in the formula (10), the rest of the substituents other than hydroxy group are each independently a hydrogen atom, a halogen atom, or an alkyl group, preferably a hydrogen atom.

### (Step 1)

Step 1 is a step of reacting a compound represented by the formula (7) using potassium tert-butoxide and tert-butyl alcohol in the presence of an aprotic solvent to obtain a compound represented by the formula (11).

When the amount of potassium tert-butoxide used in step 1 is too large, stirring becomes difficult, whereas when the amount is too small, the reaction does not proceed. Therefore, the amount of potassium tert-butoxide to be used is preferably 1.0 to 5.0 equivalents, more preferably 1.5 to 4.0 equivalents, with respect to the compound represented by the formula (7).

When the amount of tert-butyl alcohol to be used in step 1 is too large, the reaction system becomes diluted and the reaction does not proceed, whereas when the amount is too small, stirring becomes difficult. Therefore, the amount of tert-butyl alcohol to be used is preferably 5 to 20 times by weight, more preferably 7 to 10 times by weight, with respect to the compound represented by the formula (7).

The aprotic solvent to be used in step 1 is used for the purpose of preventing solidification of tert-butyl alcohol, and therefore must be compatible with tert-butyl alcohol, have a melting point of 0°C or lower, and be non-reactive. Specifically, benzene and toluene are preferred, and toluene is more preferred.

When the amount of aprotic solvent to be used in step 1 is too large, the reaction system becomes diluted and the reaction does not proceed, whereas when the amount is too small, tert-butyl alcohol solidifies and stirring becomes difficult. Therefore, the amount of aprotic solvent to be used is preferably 1 to 10 times by weight, more preferably 1 to 2 times by weight, with respect to the compound represented by the formula (7).

The reaction temperature in step 1 is not particularly limited as long as the reaction system does not solidify and can be controlled to a temperature equal to or lower than the boiling point of the reaction solvent. It is preferably 0 to 80°C, more preferably 10 to 60°C. The reaction time is not particularly limited, and is preferably 1 to 72 hr, more preferably 1 to 24 hr.

After the reaction in step 1, purification can be performed by a general known technique without requiring a purification step with low productivity such as column chromatography. Specifically, washing with water to remove hydrophilic impurities, adsorption purification with an ion exchange resin, concentration to remove the solvent and the like can be performed.

### (Step 2)

Step 2 is a step of reacting the compound represented by the formula (11) with a compound represented by the formula (13) using a condensing agent to obtain a compound represented by the formula (12).

The condensing agent to be used in step 2 is not limited as long as it is a reagent used in a general esterification reaction, and a carbodiimide-based condensing agent capable of reacting under mild conditions is preferred. Specifically, it is a condensing agent selected from the group consisting of 1-(3-dimethylaminopropyl)-3-ethylcarbodiimide hydrochloride (EDC hydrochloride), 1-(3-dimethylaminopropyl)-3-ethylcarbodiimide (EDC), N,N'-dicyclohexylcarbodiimide (DCC), and N,N'-diisopropylcarbodiimide (DIC), more preferably EDC hydrochloride. The amount of the condensing agent to be used in step 2 is preferably 0.9 to 3.0 equivalents, more preferably 1.0 to 2.0 equivalents, with respect to the hydroxy group of the compound represented by the formula (11). In addition, in the reaction of step 2, 4-dimethylaminopyridine (DMAP), triethylamine, triisopropylamine, tributylamine, and the like can be added as an additive. The amount of the additive to be used is preferably 0.1 to 0.5 equivalents, more preferably 0.1 to 0.3 equivalents, with respect to the hydroxy group of the compound represented by the formula (11).

The solvent to be used in step 2 is not particularly limited as long as it is an aprotic solvent in which the raw materials can be dissolved. Specific examples include chloroform, dichloromethane, ethyl acetate, and toluene, preferably chloroform and dichloromethane. The amount of the solvent to be used in step 2 is preferably 3 to 50 times by weight, more preferably 5 to 20 times by weight, with respect to the compound represented by the formula (11).

The reaction temperature in step 2 is not particularly limited, and is preferably 0 to 80°C, more preferably 10 to 30°C. The reaction time is not particularly limited, and is preferably 1 to 72 hr, and more preferably 1 to 24 hr.

After the reaction in step 2, purification can be performed by a general known technique without requiring a purification step with low productivity such as column chromatography. Specifically, washing with water to remove hydrophilic impurities, adsorption purification with an ion exchange resin, concentration to remove the solvent and the like can be performed.

### (Step 3)

Step 3 is a step of deprotecting the tert-butyl group of the compound represented by the formula (12) by using trifluoromethanesulfonic acid, methanesulfonic acid, a combination of trimethylsilyl chloride (TMS-Cl) and sodium iodide, or trimethylsilyl iodide (TMS-I) in an organic solvent to obtain a compound represented by the formula (1).

The organic solvent used in step 3 is not particularly limited as long as it dissolves the compound represented by the formula (12) and can be easily evaporated under reduced pressure. Specifically, acetonitrile, dichloromethane, chloroform, and the like can be used, and a single solvent or a mixture of two or more solvents can be used.

The amount of the organic solvent to be used in step 3 is preferably 2 to 30 times by weight, more preferably 5 to 20 times by weight, with respect to the formula (12). When the amount of the organic solvent is too large, the reaction system is diluted and the reaction does not proceed, whereas when the amount is too small, the aryl ester may be decomposed by the reagent.

In step 3, the tert-butyl group of the compound represented by the formula (12) is deprotected using any one of trifluoromethanesulfonic acid, methanesulfonic acid, a combination of TMS-Cl and sodium iodide, or TMS-I.

When R²⁰ in the formula (5) is a saturated aliphatic hydrocarbon group in I, or when R²⁰ in the formula (5) is II and the aliphatic hydrocarbon group of R²¹ in the formula (6) is saturated, any of trifluoromethanesulfonic acid, methanesulfonic acid, a combination of TMS-Cl and sodium iodide, or TMS-I may be used without any problem.

When R²⁰ in the formula (5) is an unsaturated aliphatic hydrocarbon group in I, or when R²⁰ in the formula (5) is II, and when the aliphatic hydrocarbon group of R²¹ in the formula (6) is unsaturated, the combination of TMS-Cl and sodium iodide, or TMS-I cannot be used because they react with unsaturated bonds. Therefore, either trifluoromethanesulfonic acid or methanesulfonic acid is used.

The amount of trifluoromethanesulfonic acid, methanesulfonic acid, a combination of TMS-Cl and sodium iodide, or TMS-I to be used in step 3 is preferably 0.5 to 6 equivalents, more preferably 1 to 4 equivalents, with respect to the formula (12). When the amount of the reagent is too small, the reaction does not proceed, whereas when the amount is too large, the aryl ester may be decomposed by the reagent.

The reaction temperature in step 3 is preferably 10 to 40°C, more preferably 20 to 30°C. When the temperature is too low, the reaction does not proceed, whereas when the temperature is too high, the aryl ester may decompose. The reaction time is not particularly limited, and is preferably 1 to 72 hr, more preferably 1 to 24 hr.

After the reaction in step 3, purification can be performed by a general known technique. Specifically, washing with water to remove hydrophilic impurities, adsorption purification with an ion exchange resin, purification using crystallization, concentration to remove the solvent and the like can be performed.

The compound represented by the formula (7) and the compound represented by the formula (13) to be used in the production method of the present invention are commercially available or can be produced according to a method known per se.

### [Production method of cationic lipid]

Next, a method for producing a cationic lipid is described. The method for producing a cationic lipid of the present invention includes the (step 1), (step 2) and (step 3) in the aforementioned method for producing aryl ester-containing carboxylic acid described above, and (step 4) a step of condensing the obtained aryl ester-containing carboxylic acid represented by the formula (1) and a compound having a disulfide bond, at least one tertiary nitrogen, and at least one hydroxy group or amino group. The number of tertiary nitrogens is preferably 1 to 4, more preferably 1 or 2. The number of hydroxyl or amino groups is preferably 1 to 3, more preferably 1 or 2.

The compound to be used in step 4 is preferably a compound represented by the following formula (14).

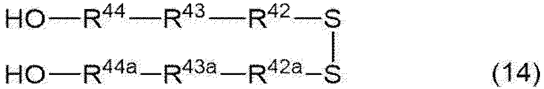

In the formula (14), R⁴² and R^{42a} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group and the like. Preferably, R⁴² and R^{42a} are each independently methylene group, ethylene group, trimethylene group, isopropylene group or tetramethylene group, most preferably ethylene group. R⁴² may be the same as or different from R^{42a}, and R⁴² is preferably the same group as R^{42a}.

R⁴³ and R^{43a} are each independently a non-cyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the non-cyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, t-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like, preferably methyl group, ethyl group, propyl group or isopropyl group, most preferably methyl group.

A preferred specific structure of the non-cyclic alkyl tertiary amino group having 1 to 6 carbon atoms and one tertiary amino group is represented by C¹.

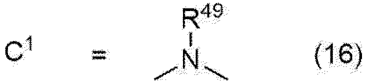

R⁴⁹ in C¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, t-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. R⁴⁹ is preferably methyl group, ethyl group, propyl group or isopropyl group, most preferably methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically aziridylene group, azetidylene group, pyrrolidylene group, piperidylene group, imidazolidylene group, or piperazylene group, preferably pyrrolidylene group, piperidylene group, or piperazylene group, most preferably piperidylene group.

A preferred specific structure of the cyclic alkylene tertiary amino group having 2 to 5 carbon atoms and one tertiary amino group is represented by C².

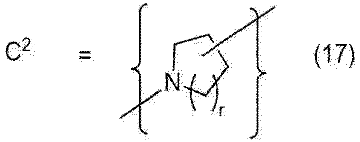

The r of C² is 1 or 2. When r is 1, C² is a pyrrolidylene group, and when r is 2, C² is a piperidylene group.

A preferred specific structure of the cyclic alkylene tertiary amino group having 2 to 5 carbon atoms and two tertiary amino groups is represented by C³.

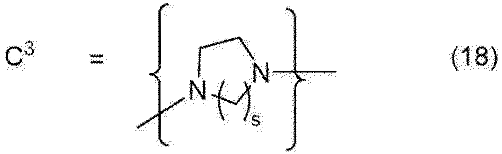

The s of C³ is 1 or 2. When s is 1, C³ is an imidazolidylene group, and when s is 2, C³ is a piperazylene group.

R⁴³ may be the same as or different from R^{43a}, and R⁴³ is preferably the same group as R^{43a}.

R⁴⁴ and R^{44a} are each independently an alkylene group or an oxydialkylene group each having not more than 8 carbon atoms, preferably each independently an alkylene group having not more than 8 carbon atoms.

The alkylene group having not more than 8 carbon atoms shown by R⁴⁴ or R^{44a} may be linear or branched, preferably linear. The number of carbons contained in the alkylene group is preferably not more than 6, most preferably not more than 4. Specific examples of the alkylene group having not more than 8 carbon atoms include methylene group, ethylene group, propylene group, isopropylene group, tetramethylene group, isobutylene group, pentamethylene group, hexamethylene group, heptamethylene group, octamethylene group and the like, preferably methylene group, ethylene group, propylene group, and tetramethylene group, most preferably ethylene group.

The oxydialkylene group having not more than 8 carbon atoms shown by R⁴⁴ or R^{44a} contains alkylene groups via an ether bond (alkylene-O-alkylene), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydipropylene group, oxydibutylene group and the like. Preferably, it is oxydimethylene group, oxydiethylene group, or oxydipropylene group, most preferably oxydiethylene group.

R⁴⁴ may be the same as or different from R^{44a}, and R⁴⁴ is preferably the same group as R^{44a}.

When the compound having a disulfide bond, at least one tertiary nitrogen, and at least one hydroxyl group or amino group to be used in step 4 is a compound represented by the formula (14), the cationic lipid is a compound represented by the formula (20).

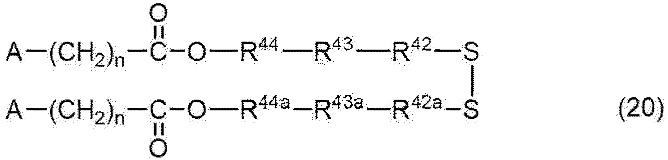

In the formula (20), each symbol is as defined above.

Specific examples of the cationic lipid include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, and O-Ph-C3M.

**[Table 1-1]**

| name of cationic lipid | structure |
|---|---|
| O-Ph-P3C1 | 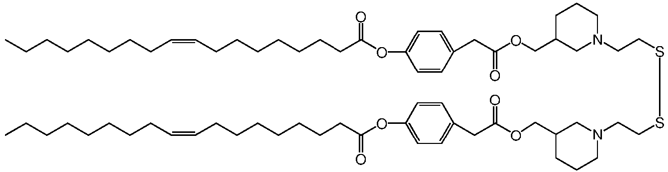 |
| O-Ph-P4C1 | 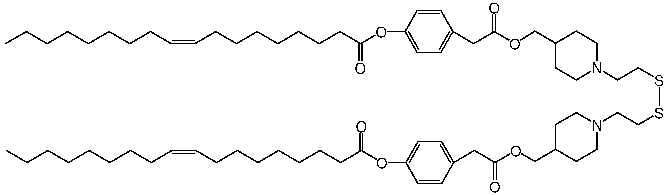 |
| O-Ph-P4C2 (or SS-OP) | 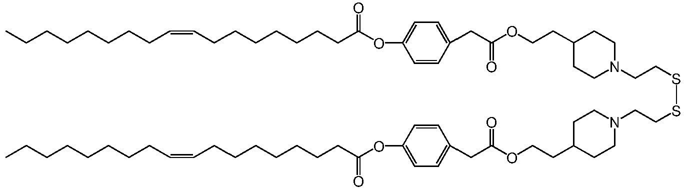 |
| O-Bn-P4C2 | 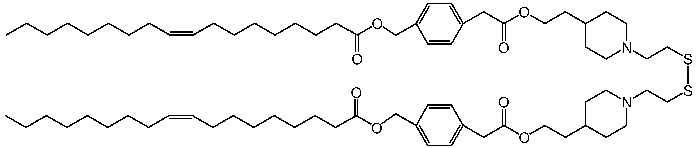 |
| E-Ph-P4C2 (or SS-EP) | 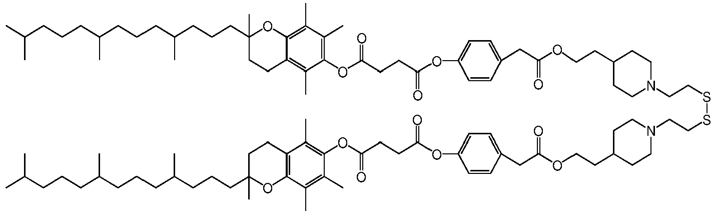 |

**[Table 1-2]**

| name of cationic lipid | structure |
|---|---|
| L-Ph-P4C2 | 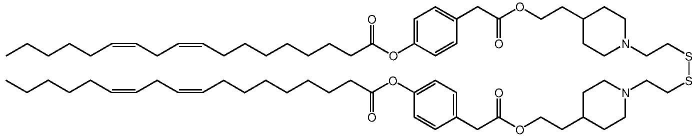 |
| HD-Ph-P4C2 | 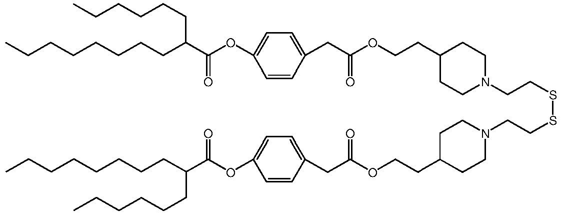 |
| O-Ph-amide-P4C2 | 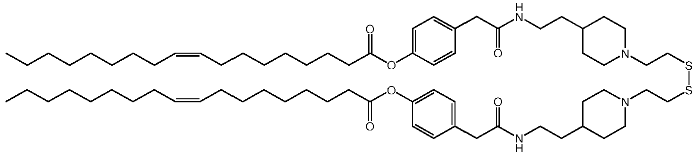 |
| O-Ph-C3M | 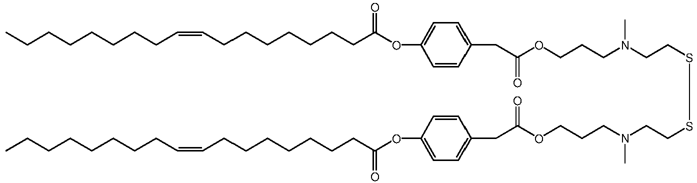 |

Step 4 is a step of condensing aryl ester-containing carboxylic acid represented by the formula (1) and a compound having a disulfide bond, at least one tertiary nitrogen, and at least one hydroxy group or amino group. In a preferred embodiment, step 4 is a step of condensing aryl ester-containing carboxylic acid represented by the formula (1) with hydroxy groups of a compound represented by the formula (14) described above.

There is no limitation on the condensation reaction used in step 4 as long as a commonly known technique is used, but the reaction is preferably performed using a condensing agent.

The condensing agent to be used in step 4 is not limited as long as it is a reagent used in a general esterification reaction, and a carbodiimide-based condensing agent capable of reacting under mild conditions is preferred. Specifically, it is a condensing agent selected from the group consisting of 1-(3-dimethylaminopropyl)-3-ethylcarbodiimide hydrochloride (EDC hydrochloride), 1-(3-dimethylaminopropyl)-3-ethylcarbodiimide (EDC), N,N'-dicyclohexylcarbodiimide (DCC), and N,N'-diisopropylcarbodiimide (DIC), more preferably EDC hydrochloride or EDC. The amount of the condensing agent to be used in step 4 is preferably 0.9 to 3.0 equivalents, more preferably 1.0 to 2.0 equivalents, with respect to the hydroxy group of the compound represented by the formula (14). In addition, in the reaction of step 4, 4-dimethylaminopyridine (DMAP), triethylamine, triisopropylamine, tributylamine, and the like can be added as an additive. The amount of the additive to be used is preferably 0.1 to 0.5 equivalents, more preferably 0.1 to 0.3 equivalents, with respect to the hydroxy group of the compound represented by the formula (14).

The solvent to be used in step 4 is not particularly limited as long as it is an aprotic solvent in which the raw materials can be dissolved. Specific examples include chloroform, dichloromethane, ethyl acetate, and toluene, preferably chloroform and dichloromethane. The amount of the solvent to be used in step 4 is preferably 3 to 50 times by weight, more preferably 5 to 40 times by weight, with respect to the compound represented by the formula (14).

The reaction temperature in step 4 is not particularly limited, and is preferably 0 to 80°C, more preferably 10 to 30°C. The reaction time is not particularly limited, and is preferably 1 to 72 hr, and more preferably 1 to 24 hr.

After the reaction in step 4, purification can be performed by a general known technique. As one embodiment, washing with water to remove hydrophilic impurities, adsorption purification with an ion exchange resin, concentration to remove the solvent and the like can be performed.

The compound represented by the formula (14) to be used in step 4 can be produced by a known method (for example, the methods described in US 2014/0335157A1 and WO 2016/121942A1).

### [Example]

The Examples of the present invention are explained in further detail in the following, but the present invention is not limited in any way by the Examples.

The room temperature in the Examples generally means about 10 to 30°C, but is not strictly limited thereto.

### [Example 1]

### Step 1: Synthesis of tert-butyl 4-hydroxyphenylacetate

Potassium tert-butoxide (manufactured by KANTO CHEMICAL CO., INC.) (338 g, 3.01 mol) was dissolved in tert-butyl alcohol (1600 g) and toluene (400 g). A solution of methyl 4-hydroxyphenylacetate (manufactured by Tokyo Chemical Industry Co., Ltd.) (250 g, 1.50 mol) in tert-butyl alcohol (400 g) and toluene (100 g) was added and the mixture was reacted at room temperature for 16 hr. A solution of phosphoric acid (400 g) in methanol (1000 g) was added to neutralize the reaction solution and the precipitated salt was filtered off. The filtrate was concentrated in an evaporator. To the concentrate was added 5% sodium hydrogen carbonate aqueous solution (1313 g) and the mixture was extracted twice with dichloromethane (1250 g). The dichloromethane layers obtained by the two extractions were collected and mixed, and then 50 g of magnesium oxide-aluminum oxide adsorbent KW-2000 (manufactured by Kyowa Chemical Industry Co., Ltd.) was added and the mixture was stirred at room temperature for 30 min. After filtering off KW-2000, the obtained solution was concentrated in an evaporator. To the concentrate was added 750 g of toluene and concentrated again in an evaporator. This operation was repeated twice, and the obtained crystals were vacuum dried to obtain tert-butyl 4-hydroxyphenylacetate (225 g, yield 72 mol%/methyl 4-hydroxyphenylacetate).

¹H-NMR spectrum (600 MHz, CD₃OD) of tert-butyl 4-hydroxyphenylacetate δ 1.42 (s, 9H), δ 3.40 (s, 2H), δ 4.85 (s, 1H), δ 6.70-6.73 (d, 2H), δ 6.70-7.06 (d, 2H)

### Step 2: Synthesis of tert-butyl 4-oleoyloxyphenylacetate

tert-Butyl 4-hydroxyphenylacetate (225 g, 1.08 mol) was dissolved in chloroform (2250 g), oleic acid (manufactured by NOF CORPORATION) (299 g, 1.06 mol), DMAP (manufactured by KOEI CHEMICAL CO., LTD.) (26 g, 0.22 mol) and EDC hydrochloride (manufactured by Tokyo Chemical Industry Co., Ltd.) (269 g, 1.40 mol) were added and the mixture was reacted at room temperature for 1 hr. The solution after the reaction was washed once with 0.1 mol/L hydrochloric acid (2250 g), once with 5% sodium hydrogen carbonate aqueous solution (2250 g), and once with ion exchange water (2250 g), and concentrated in an evaporator. Hexane (2250 g) was added to the concentrate, silica gel PSQ100B (manufactured by Fuji Silysia Chemical Ltd.) (45 g) was added and the mixture was stirred at room temperature for 30 min. PSQ100B was filtered off and the filtrate was concentrated in an evaporator, and the concentrate was vacuum dried to give tert-butyl 4-oleoyloxyphenylacetate (486 g, yield 95 mol%/tert-butyl 4-hydroxyphenylacetate).

¹H-NMR spectrum (600 MHz, CDCl₃) of tert-butyl 4-oleoyloxyphenylacetate δ 0.87-0.89 (t, 3H), δ 1.20-1.57 (m, 29H), δ 1.72-1.77 (m, 2H), δ 2.00-2.10 (m, 4H), δ 2.52-2.55 (t, 2H), δ 3.50 (s, 2H), δ 5.30-5.40 (m, 2H), δ 7.02-7.30 (d, 2H), δ 7.26-7.28 (d, 2H)

### Step 3: Synthesis of 4-oleoyloxyphenylacetic acid

tert-Butyl 4-oleoyloxyphenylacetate (298 g, 0.63 mol) was dissolved in acetonitrile (1192 g) and chloroform (1192 g), a solution of methanesulfonic acid (96.9 g, 1.01 mol) in acetonitrile (298 g) and chloroform (298 g) was added, and the mixture was reacted at room temperature for 2 hr. The solution after the reaction was washed three times with ion exchange water (1490 g) and concentrated in an evaporator. The crude product after concentration was recrystallized from hexane (894 g), and the crystals were collected to give 4-oleoyloxyphenylacetic acid (159 g, yield 61 mol%/tert-butyl 4-oleoyloxyphenylacetate) (yield: 59 mol%/oleic acid).

¹H-NMR spectrum (600 MHz, CDCl₃) of 4-oleoyloxyphenylacetic acid δ 0.89-0.91 (t, 3H), δ 1.20-1.57 (m, 20H), δ 1.72-1.85 (m, 2H), δ 2.00-2.10 (m, 4H), δ 2.52-2.70 (t, 2H), δ 3.66 (s, 2H), δ 5.30-5.40 (m, 2H), δ 7.02-7.30 (d, 2H), δ 7.28-7.32 (d, 2H)

### [Example 2]

### Step 1: Synthesis of tert-butyl 4-hydroxyphenylacetate

The title compound was synthesized by the method described in Example 1, step 1.

### Step 2: Synthesis of tert-butyl 4-(2-hexadecyloctadecanoyloxy)phenylacetate

tert-Butyl 4-hydroxyphenylacetate (1.3 g, 6.2 mmol)was dissolved in chloroform (13.2 g), 2-hexadecyloctadecanoic acid (manufactured by Tokyo Chemical Industry Co., Ltd.) (3.0 g, 5.9 mmol), DMAP (manufactured by KOEI CHEMICAL CO., LTD.) (148 mg, 1.3 mmol) and EDC hydrochloride (manufactured by Tokyo Chemical Industry Co., Ltd.) (1.3 g, 6.8 mmol) were added and the mixture was reacted at room temperature for 2 hr. The solution after the reaction was washed once with 0.1 mol/L hydrochloric acid (13.0 g), once with 5% sodium hydrogen carbonate aqueous solution (13.0 g), and once with ion exchange water (13.0 g) and concentrated in an evaporator. To the concentrate was added hexane (13 g), then PSQ100B (manufactured by Fuji Silysia Chemical Ltd.) (260 mg) was added and the mixture was stirred at room temperature for 30 min. PSQ100B was filtered off, the filtrate was concentrated in an evaporator, and the concentrate was vacuum dried to give tert-butyl 4-(2-hexadecyloctadecanoyloxy)phenylacetate (3.7 g, yield: 86 mol%/tert-butyl 4-hydroxyphenylacetate).

¹H-NMR spectrum (600 MHz, CDCl₃) of tert-butyl 4-(2-hexadecyloctadecanoyloxy)phenylacetate δ 0.87-0.90 (t, 6H), δ 1.10-1.90 (m, 69H), δ 2.50-2.60 (m, 1H), δ 3.51 (s, 2H), δ 7.00-7.01 (d, 2H), δ 7.26-7.28 (d, 2H)

### Step 3: Synthesis of 4-(2-hexadecyloctadecanoyloxy)phenylacetic acid

tert-Butyl 4-(2-hexadecyloctadecanoyloxy)phenylacetate (3.7 g, 5.3 mmol) was dissolved in chloroform (29.3 g), a solution of methanesulfonic acid (0.8 g, 8.3 mmol) in chloroform (7.3 g) was added and the mixture was reacted at room temperature for 3 hr. The solution after the reaction was washed three times with ion exchange water (36.6 g) and concentrated in an evaporator. The crude product after concentration was recrystallized from hexane (18.6 g), and the crystals were collected to give 4-(2-hexadecyloctadecanoyloxy)phenylacetic acid (2.9 g, yield: 84 mol%/tert-butyl 4-(2-hexadecyloctadecanoyloxy)phenylacetate).

¹H-NMR spectrum (600 MHz, CDCl₃) of 4-(2-hexadecyloctadecanoyloxy)phenylacetic acid δ 0.87-0.90 (t, 6H), δ 1.10-1.90 (m, 60H), δ 2.50-2.60 (m, 1H), δ 3.51 (s, 2H), δ 7.00-7.01 (d, 2H), δ 7.26-7.28 (d, 2H)

### [Example 3]

### Step 1: Synthesis of tert-butyl 4-hydroxyphenylacetate

The title compound was synthesized by the method described in Example 1, step 1.

### Step 2: Synthesis of tert-butyl 4-(D-α-tocopherol hemisuccinyloxy)phenylacetate

tert-Butyl 4-hydroxyphenylacetate (1.0 g, 4.8 mmol) was dissolved in chloroform (20.0 g), D-α-tocopherol succinate (manufactured by Tokyo Chemical Industry Co., Ltd.) (2.5 g, 4.7 mmol), DMAP (manufactured by KOEI CHEMICAL CO., LTD.) (120 mg, 1.0 mmol) and EDC hydrochloride (manufactured by Tokyo Chemical Industry Co., Ltd.) (1.2 g, 6.2 mmol) were added and the mixture was reacted at room temperature for 2 hr. The solution after the reaction was washed once with 0.1 mol/L hydrochloric acid (20 g), once with 5% sodium hydrogen carbonate aqueous solution (20 g), and once with ion exchange water (20 g) and concentrated in an evaporator. To the concentrate was added hexane (20 g), then PSQ100B (manufactured by Fuji Silysia Chemical Ltd.) (200 mg) was added, and the mixture was stirred at room temperature for 30 min. PSQ100B was filtered off, the filtrate was concentrated in an evaporator, and the concentrate was vacuum dried to give tert-butyl 4-(D-α-tocopherol hemisuccinyloxy)phenylacetate (2.5 g, yield: 80 mol%/tert-butyl 4-hydroxyphenylacetate).

¹H-NMR spectrum (600 MHz, CDCl₃) of tert-butyl 4-(D-α-tocopherol hemisuccinyloxy)phenylacetate δ 0.80-0.90 (t, 12H), δ 1.0-1.9 (m, 35H), δ 1.96 (s, 3H)δ 2.00 (s, 3H), δ 2.08 (s, 3H), δ 2.55-2.60 (t, 2H), δ 2.81-2.84 (t, 2H), δ 2.91-2.94 (t, 2H), δ 3.50 (s, 2H), δ 7.02-7.30 (d, 2H), δ 7.26-7.28 (d, 2H)

### Step 3: Synthesis of 4-(D-α-tocopherol hemisuccinyloxy)phenylacetic acid

tert-Butyl 4-(D-α-tocopherol hemisuccinyloxy)phenylacetate (1.4 g, 2.0 mmol) was dissolved in chloroform (12.0 g), a solution of methanesulfonic acid (0.3 g, 3.2 mmol) in chloroform (2.0 g) was added and the mixture was reacted at room temperature for 3 hr. The solution after the reaction was washed three times with ion exchange water (12.1 g) and concentrated in an evaporator. The crude product after concentration was recrystallized from hexane (4.5 g), and the crystals were collected to give 4-(D-α-tocopherol hemisuccinyloxy)phenylacetic acid (0.6 g, yield: 55 mol%/tert-butyl 4-(D-α-tocopherol hemisuccinyloxy)phenylacetate).

¹H-NMR spectrum (600 MHz, CDCl₃) of 4-(D-α-tocopherol hemisuccinyloxy)phenylacetic acid δ 0.80-0.90 (t, 12H), δ 1.0-1.9 (m, 26H), δ 1.96 (s, 3H)δ 2.00 (s, 3H), δ 2.08 (s, 3H), δ 2.55-2.60 (t, 2H), δ 2.81-2.84 (t, 2H), δ 2.91-2.94 (t, 2H), δ 3.66 (s, 2H), δ 7.02-7.30 (d, 2H), δ 7.28-7.32 (d, 2H)

### [Example 4]

### Step 1: Synthesis of tert-butyl 4-hydroxyphenylacetate

The title compound was synthesized by the method described in Example 1, step 1.

### Step 2: Synthesis of tert-butyl 4-linoleoyloxyphenylacetate

tert-Butyl 4-hydroxyphenylacetate (1.0 g, 4.8 mmol) was dissolved in chloroform (10.0 g), linoleic acid (manufactured by NOF CORPORATION) (1.0 g, 4.7 mmol), DMAP (manufactured by KOEI CHEMICAL CO., LTD.) (120 mg, 1.0 mmol) and EDC hydrochloride (manufactured by Tokyo Chemical Industry Co., Ltd.) (1.2 g, 6.2 mmol) were added and the mixture was reacted at room temperature for 2 hr. The solution after the reaction was washed once with 0.1 mol/L hydrochloric acid (10 g), once with 5% sodium hydrogen carbonate aqueous solution (10 g), and once with ion exchange water (10 g) and concentrated in an evaporator. To the concentrate was added hexane (10 g), then PSQ100B (manufactured by Fuji Silysia Chemical Ltd.) (200 mg) was added, and the mixture was stirred at room temperature for 30 min. PSQ100B was filtered off, the filtrate was concentrated in an evaporator, and the concentrate was vacuum dried to give tert-butyl 4-linoleoyloxyphenylacetate (1.8 g, yield: 80 mol%/tert-butyl 4-hydroxyphenylacetate).

¹H-NMR spectrum (600 MHz, CDCl₃) of tert-butyl 4-linoleoyloxyphenylacetate δ 0.87-0.90 (t, 3H), δ 1.20-1.40 (m, 23H), δ 1.55-1.62 (m, 2H), δ 1.97-2.07 (m, 4H), δ 2.27-2.30 (t, 2H), δ 2.76-2.78 (t, 2H), δ 3.50 (s, 2H), δ 5.32-5.39 (m, 4H), δ 7.02-7.30 (d, 2H), δ 7.26-7.28 (d, 2H)

### Step 3: Synthesis of 4-linoleoyloxyphenylacetic acid

tert-Butyl 4-linoleoyloxyphenylacetate (1.0 g, 2.0 mmol) was dissolved in acetonitrile (4.0 g) and chloroform (4.0 g), a solution of methanesulfonic acid (0.3 g, 3.2 mmol) in acetonitrile (1.0 g) and chloroform (1.0 g) was added, and the mixture was reacted at room temperature for 3 hr. The solution after the reaction was washed three times with ion exchange water (10.0 g) and concentrated in an evaporator. The crude product after concentration was recrystallized from hexane (3 g) and crystals were collected to give 4-linoleoyloxyphenylacetic acid (0.5 g, yield: 60 mol%/tert-butyl 4-linoleoyloxyphenylacetate).

¹H-NMR spectrum (600 MHz, CDCl₃) of 4-linoleoyloxyphenylacetic acid δ 0.87-0.90 (t, 3H), δ 1.20-1.40 (m, 14H), δ 1.55-1.62 (m, 2H), δ 1.97-2.07 (m, 4H), δ 2.27-2.30 (t, 2H), δ 2.76-2.78 (t, 2H), δ 3.66 (s, 2H), δ 5.32-5.39 (m, 4H), δ 7.02-7.30 (d, 2H), δ 7.28-7.32 (d, 2H)

### [Example 5]

### Step 1: Synthesis of tert-butyl 4-hydroxyphenylacetate

The title compound was synthesized by the method described in Example 1, step 1.

### Step 2: Synthesis of tert-butyl 4-(2-hexyldecanoyloxy)phenylacetate

tert-Butyl 4-hydroxyphenylacetate (1.0 g, 4.8 mmol) was dissolved in chloroform (10.0 g), 2-hexyldecanoic acid (manufactured by Tokyo Chemical Industry Co., Ltd.) (1.2 g, 4.7 mmol), DMAP (manufactured by KOEI CHEMICAL CO., LTD.) (120 mg, 1.0 mmol) and EDC hydrochloride (manufactured by Tokyo Chemical Industry Co., Ltd.) (1.2 g, 6.2 mmol) were added and the mixture was reacted at room temperature for 2 hr. The solution after the reaction was washed once with 0.1 mol/L hydrochloric acid (10 g), once with 5% sodium hydrogen carbonate aqueous solution (10 g), and once with ion exchange water (10 g) and concentrated in an evaporator. To the concentrate was added hexane (10 g), then PSQ100B (manufactured by Fuji Silysia Chemical Ltd.) (200 mg) was added and the mixture was stirred at room temperature for 30 min. PSQ100B was filtered off, the filtrate was concentrated in an evaporator, and the concentrate was vacuum dried to give tert-butyl 4-(2-hexyldecanoyloxy)phenylacetate (1.7 g, yield: 80 mol%/tert-butyl 4-hydroxyphenylacetate).

¹H-NMR spectrum (600 MHz, CDCl₃) of tert-butyl 4-(2-hexyldecanoyloxy)phenylacetate δ 0.77-0.89 (t, 6H), δ 1.27-1.42 (m, 29H), δ 1.71-1.77 (m, 4H), δ 2.52-2.56 (m, 1H), δ 3.50 (s, 2H), δ 7.02-7.30 (d, 2H), δ 7.26-7.28 (d, 2H)

### Step 3: Synthesis of 4-(2-hexyldecanoyloxy)phenylacetic acid

tert-Butyl 4-(2-hexyldecanoyloxy)phenylacetate (1.0 g, 2.2 mmol) was dissolved in acetonitrile (4.0 g) and chloroform (4.0 g), a solution of methanesulfonic acid (0.3 g, 3.6 mmol) in acetonitrile (1.0 g) and chloroform (1.0 g) was added and the mixture was reacted at room temperature for 3 hr. The solution after the reaction was washed three times with ion exchange water (10.0 g) and concentrated in an evaporator. The crude product after concentration was recrystallized from hexane (3 g) and the crystals were collected to give 4-(2-hexyldecanoyloxy)phenylacetic acid (0.5 g, yield: 55 mol%/tert-butyl 4-(2-hexyldecanoyloxy)phenylacetate).

¹H-NMR spectrum (600 MHz, CDCl₃) of 4-(2-hexyldecanoyloxy)phenylacetic acid δ 0.77-0.89 (t, 6H), δ 1.27-1.42 (m, 20H), δ 1.71-1.77 (m, 4H), δ 2.52-2.56 (m, 1H), δ 3.64 (s, 2H), δ 7.03-7.06 (m, 2H), δ 7.28-7.31 (m, 2H)

### [Example 6]

### Step 1: Synthesis of tert-butyl 4-hydroxyphenylacetate

The title compound was synthesized by the method described in Example 1, step 1.

### Step 2: Synthesis of tert-butyl 4-(9-(heptadecan-9-yloxy)-9-oxononanoyloxy)phenylacetate

tert-Butyl 4-hydroxyphenylacetate (1.0 g, 4.8 mmol) was dissolved in chloroform (10.0 g), 9-(heptadecan-9-yloxy)-9-oxononanoic acid (synthesized by the method described in Patent Literature 3) (2.0 g, 4.7 mmol), DMAP (manufactured by KOEI CHEMICAL CO., LTD.) (120 mg, 1.0 mmol) and EDC hydrochloride (manufactured by Tokyo Chemical Industry Co., Ltd.) (1.2 g, 6.2 mmol) were added, and the mixture was reacted at room temperature for 2 hr. The solution after the reaction was washed once with 0.1 mol/L hydrochloric acid (15 g), once with 5% sodium hydrogen carbonate aqueous solution (15 g), and once with ion exchange water (15 g) and concentrated in an evaporator. To the concentrate was added hexane (15 g), then PSQ100B (manufactured by Fuji Silysia Chemical Ltd.) (200 mg) was added, and the mixture was stirred at room temperature for 30 min. PSQ100B was filtered off, the filtrate was concentrated in an evaporator, and the concentrate was vacuum dried to give tert-butyl 4-(9-(heptadecan-9-yloxy)-9-oxononanoyloxy)phenylacetate (2.4 g, yield 80 mol%/tert-butyl 4-hydroxyphenylacetate).

¹H-NMR spectrum (600 MHz, CDCl₃) of tert-butyl 4-(9-(heptadecan-9-yloxy)-9-oxononanoyloxy)phenylacetate δ 0.84-0.90 (t, 6H), δ 1.20-1.40 (m, 39H), δ 1.55-1.70 (m, 4H), δ 1.40-1.50 (m, 4H), δ 2.25-2.34 (m, 4H), δ 4.80-4.90 (m, 1H), δ 3.50 (s, 2H), δ 7.02-7.30 (d, 2H), δ 7.26-7.28 (d, 2H)

### Step 3: Synthesis of 4-(9-(heptadecan-9-yloxy)-9-oxononanoyloxy)phenylacetic acid

tert-Butyl 4-(9-(heptadecan-9-yloxy)-9-oxononanoyloxy)phenylacetate (1.2 g, 2.0 mmol) was dissolved in chloroform (10.0 g), a solution of methanesulfonic acid (0.3 g, 3.2 mmol) in chloroform (2.0 g) was added, and the mixture was reacted at room temperature for 3 hr. The solution after the reaction was washed three times with ion exchange water (14.0 g) and concentrated in an evaporator. The crude product after concentration was recrystallized from hexane (3.6 g), and the crystals were collected to give 4-(9-(heptadecan-9-yloxy)-9-oxononanoyl)oxyphenylacetic acid (0.6 g, yield 55 mol%/tert-butyl 4-(9-(heptadecan-9-yloxy)-9-oxononanoyloxy)phenylacetate).

¹H-NMR spectrum (600 MHz, CDCl₃) of 4-(9-(heptadecan-9-yloxy)-9-oxononanoyloxy)phenylacetic acid δ 0.84-0.90 (t, 6H), δ 1.20-1.40 (m, 30H), δ 1.55-1.70 (m, 4H), δ 1.40-1.50 (m, 4H), δ 2.25-2.34 (m, 4H), δ 4.80-4.90 (m, 1H), δ 3.66 (s, 2H), δ 7.02-7.30 (d, 2H), δ 7.28-7.32 (d, 2H)

### [Example 7]

### Step 1: Synthesis of tert-butyl 4-hydroxyphenylacetate

Potassium tert-butoxide (manufactured by KANTO CHEMICAL CO., INC.) (6077 g, 54.2 mol) was dissolved in tert-butyl alcohol (19.20 kg) and toluene (4800 g). A solution of methyl 4-hydroxyphenylacetate (manufactured by Tokyo Chemical Industry Co., Ltd.) (3000 g, 18.1 mol) in tert-butyl alcohol (4800 g) and toluene (1200 g) was added and the mixture was reacted at 40°C for 2 hr. The reaction solution was neutralized with a solution of phosphoric acid (6300 g) in methanol (12.00 kg), the precipitated salt was filtered off, and the filtrate was concentrated in an evaporator. To the concentrate was added 5% sodium hydrogen carbonate aqueous solution (15.75 kg) and the mixture was extracted twice with dichloromethane (25.50 kg). The dichloromethane layers obtained by the two extractions were collected and mixed, magnesium oxide-aluminum oxide adsorbent KW-2000 (manufactured by Kyowa Chemical Industry Co., Ltd.) (600 g) was added, and the mixture was stirred at room temperature for 30 min. KW-2000 was filtered off and the obtained solution was concentrated in an evaporator. To the concentrate was added toluene (9000 g) and the mixture was concentrated again in an evaporator. This operation was repeated twice and the obtained crystals were vacuum dried to give tert-butyl 4-hydroxyphenylacetate (2935 g, yield 78 mol%/methyl 4-hydroxyphenylacetate).

### Step 2: Synthesis of tert-butyl 4-oleoyloxyphenylacetate

tert-Butyl 4-hydroxyphenylacetate (20.0 g, 0.096 mol) was dissolved in chloroform (200 g), oleic acid (manufactured by NOF CORPORATION) (29.9 g, 0.106 mol), DMAP (2.35 g, 0.019 mol) and EDC hydrochloride (manufactured by Tokyo Chemical Industry Co., Ltd.) (23.9 g, 0.125 mol) were added and the mixture was reacted at room temperature for 1 hr. The solution after the reaction was washed once with ion exchange water (200 g), once with 0.1 mol/L hydrochloric acid (200 g) and tert-butyl alcohol (100 g), once with 5% sodium hydrogen carbonate aqueous solution (200 g), and once with ion exchange water (100 g) and concentrated in an evaporator. To the concentrate was added hexane (200 g), then silica gel PSQ100B (manufactured by Fuji Silysia Chemical Ltd.) (4 g) was added, and the mixture was stirred at room temperature for 30 min. PSQ100B was filtered off, hydrotalcite adsorbent KW-1000 (manufactured by Kyowa Chemical Industry Co., Ltd.) (20 g) was added, and the mixture was stirred at room temperature for 30 min. KW-1000 was filtered off, the filtrate was concentrated in an evaporator, and the concentrate was vacuum dried to give tert-butyl 4-oleoyloxyphenylacetate (42.7 g, yield 94 mol%/tert-butyl 4-hydroxyphenylacetate).

### Step 3: Synthesis of 4-oleoyloxyphenylacetic acid

tert-Butyl 4-oleoyloxyphenylacetate (25.0 g, 0.053 mol) was dissolved in acetonitrile (100 g), a solution of methanesulfonic acid (8.13 g, 0.085 mol) in acetonitrile (25 g) was added and the mixture was reacted at room temperature for 2 hr. To the solution after the reaction was added chloroform (125 g), and the mixture was washed three times with ion exchange water (125 g). After washing, DMAP (1.29 g, 0.011 mol) and ion exchange water (25 g) were added and the mixture was stirred for 30 min and washed once with 0.1 mol/L hydrochloric acid (125 g) and twice with ion exchange water (125 g). After concentration in an evaporator, the concentrate was recrystallized from hexane (68 g), and the crystals were collected to give 4-oleoyloxyphenylacetic acid (16.0 g, yield 73 mol%/tert-butyl 4-oleoyloxyphenylacetate).

### [Comparative Example 1]

### The method described in Patent Literature 2

### Synthesis of oleic anhydride

Oleic acid (manufactured by NOF CORPORATION) (7.0 g, 24.8 mmol) was dissolved in chloroform (56 g) at room temperature, and the mixture was cooled to 10-15°C. Thereto was added dropwise a suspension of DCC (manufactured by Osaka Synthetic Chemical Laboratories, Inc.) (2.5 g, 12.1 mmol) dissolved in chloroform (14 g), and the mixture was reacted at 10 to 25°C for 2 hr. The reaction solution was filtered, and the filtrate was concentrated in an evaporator. The obtained concentrate was re-dissolved in hexane (21 g), and insoluble material was removed by filtration. The obtained filtrate was concentrated in an evaporator to give oleic anhydride (6.0 g).

### Synthesis of 4-oleoyloxyphenylacetic acid

Oleic anhydride (4.3 g, 7.9 mmol) and 4-hydroxyphenylacetic acid (manufactured by Tokyo Chemical Industry Co., Ltd.) (0.6 g, 3.9 mmol) were dissolved in chloroform (65 g). Thereto was added DMAP (manufactured by KOEI CHEMICAL CO., LTD.) (0.2 g, 1.6 mmol) and the mixture was reacted at room temperature for 9 hr. The reaction solution was washed twice with 10% aqueous acetic acid solution (22 g) and twice with ion exchange water (22 g). Magnesium sulfate (manufactured by KANTO CHEMICAL CO., INC.) (1.3 g) was added to the organic layer, and the mixture was stirred for 30 min. Magnesium sulfate was filtered off, and the filtrate was concentrated in an evaporator. The concentrate was re-dissolved in hexane (28 g), the insoluble material was filtered off, and the filtrate was extracted 6 times with acetonitrile (168 g). The acetonitrile layer was recovered and concentrated in an evaporator to give the same 4-oleoyloxyphenylacetic acid (1.8 g) as in Example 1 (yield: 37 mol%/4-hydroxyphenylacetic acid) (yield: 24 mol%/oleic acid) (cf. yield in Example 1: 59 mol%/oleic acid).

¹H-NMR spectrum (600 MHz, CDCl₃) of 4-oleoyloxyphenylacetic acid δ 0.89-0.91 (t, 3H), δ 1.20-1.57 (m, 20H), δ 1.72-1.85 (m, 2H), δ 2.00-2.10 (m, 4H), δ 2.52-2.70 (t, 2H), δ 3.66 (s, 2H), δ 5.30-5.40 (m, 2H), δ 7.02-7.30 (d, 2H), δ 7.28-7.32 (d, 2H)

### [Analysis method]

100 µL of the solution after the reaction in Examples 1 to 7 and Comparative Example 1 was collected and mixed with 1 mL of deuterochloroform, and then ¹H-NMR measurement was performed. The integral values of all peaks from 3.5 ppm to 4.0 ppm, which correspond to the benzyl position of the 4-hydroxyphenylacetic acid structure (theoretical integral value: 2), were set to 2.000, and the integral value of the methylene peak at 3.84 ppm, which is specific to the oligomer of 4-hydroxyphenylacetic acid, was obtained to calculate the molar amount of the oligomer relative to the 4-hydroxyphenylacetic acid structure. Note that the integral value was set to detect up to 0.001, Which made the detection limit 0.05 mol% (=0.001/2.000). The results are shown in Table 2.

**[Table 2]**

| sample after reaction | oligomer amount (mol%) relative to molar quantity of 4-hydroxyphenylacetic acid |
|---|---|
| Example 1 | not detected |
| Example 2 | not detected |
| Example 3 | not detected |
| Example 4 | not detected |
| Example 5 | not detected |
| Example 6 | not detected |
| Example 7 | not detected |
| Comparative Example 1 | 10.73 |

From the above results, no oligomers were detected in the Examples, and the amount of oligomers was successfully reduced compared to Comparative Example 1. Therefore, this production method is useful as a production method of chemical substances used as pharmaceutical products and raw materials thereof.

### [Example 8]

### Step 4: Synthesis of O-Ph-P4C2

Bis{2-[4-(2-hydroxyethyl)piperidyl]ethyl}disulfide (di-4PE-form) (48.0 g, 0.13 mol) synthesized by the method described in US2014/0335157A1, 4-oleoyloxyphenylacetic acid (111.5 g, 0.27 mol) obtained by the method of Example 1, and DMAP (6.2 g, 0.05 mol) were dissolved in dichloromethane (1680 g) at room temperature. EDC (73.3 g, 0.38 mol) was added thereto, and the mixture was reacted at 20-30°C for 21 hr. The reaction solution was washed twice with ion exchange water (1200 g) and the organic layer was concentrated in an evaporator to give a crude product (161 g). The obtained crude product was column purified to give O-Ph-P4C2 (117.5 g).

¹H-NMR spectrum (600 MHz, CDCl₃) of O-Ph-P4C2 δ 0.86-0.90 (t, 6H), δ 1.22-1.42 (m, 46H), δ 1.54-1.76 (m, 12H), δ 1.94-2.03 (m, 12H), δ 2.52-2.56 (m, 4H), δ 2.62-2.66 (m, 4H), δ 2.80-2.89 (m, 8H), δ 3.59 (s, 4H), δ 4.11-4.14 (t, 4H), δ 5.34-5.37 (m, 4H), δ 7.02-7.05 (m, 4H), δ 7.26-7.30 (m, 4H)

### [Example 9]

### Step 4: Synthesis of E-Ph-P4C2

di-4PE form (0.350 g, 0.929 mmol), 4-(D-α-tocopherol hemisuccinyloxy)phenylacetic acid (1.04 g, 1.95 mmol) obtained by the method of Example 3, and DMAP (0.0454 g, 0.372 mmol) were dissolved in chloroform (10.5 g) at room temperature. Thereto was added EDC (0.534 g, 2.79 mmol), and the mixture was reacted at 30-35°C for 4 hr. The reaction solution was washed twice with 20% brine (7.00 g) and dehydrated using magnesium sulfate (0.350 g). Magnesium sulfate was filtered off, and the filtrate was concentrated in an evaporator to give a crude product (1.31 g). The obtained crude product was subjected to column purification to give E-Ph-P4C2 (0.860 g).

¹H-NMR spectrum (600 MHz, CDCl₃) of E-Ph-P4C2 δ 0.83-0.87 (m, 24H), δ 1.02-1.85 (m, 66H), δ 1.94-1.98 (m, 10H), δ 2.00 (s, 6H), δ 2.08 (s, 6H), δ 2.53-2.66 (m, 8H), δ 2.71-2.85 (m, 8H), δ 2.85-2.95 (m, 8H), δ 3.65 (s, 4H), δ 4.11-4.14 (t, 4H), δ 7.05-7.08 (m, 2H), δ 7.27-7.31 (m, 2H)

### [Industrial Applicability]

The production method of the present invention can be used as a method for producing raw materials of pharmaceutical products required to have high purity, LNP raw materials and the like. The cationic lipid produced by the production method of the present invention is useful as an LNP raw material.

This application is based on patent application No. 2022-051920 filed in Japan, the contents of which are encompassed in full herein.

## Claims

1. A method for producing an aryl ester-containing carboxylic acid represented by the following formula (1), comprising the following three steps:
(step 1) a step of reacting a compound represented by the following formula (7) using potassium tert-butoxide and tert-butyl alcohol in the presence of an aprotic solvent to obtain a compound represented by the following formula (11),
(step 2) a step of reacting the compound represented by the following formula (11) with a compound represented by the following formula (13) using a condensing agent to obtain a compound represented by the following formula (12), and
(step 3) a step of deprotecting the tert-butyl group of the compound represented by the following formula (12) by using trifluoromethanesulfonic acid, methanesulfonic acid, a combination of trimethylsilyl chloride (TMS-Cl) and sodium iodide, or trimethylsilyl iodide (TMS-I) in an organic solvent to obtain a compound represented by the following formula (1); 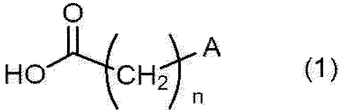
in the formula (1), A is a group represented by the following formula (2), (3), or (4), and n is an integer of 1 to 10 indicating the number of repeating units of methylene group, 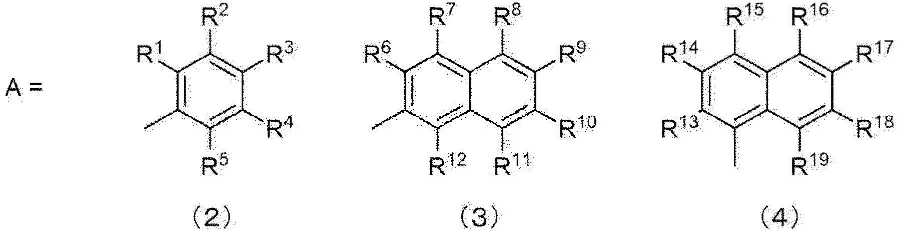
in the formula (2), at least one selected from R¹ to R⁵ is a group represented by the following formula (5), and the rest are each independently a hydrogen atom, a halogen atom, or an alkyl group,
in the formula (3), at least one selected from R⁶ to R¹² is a group represented by the following formula (5), and the rest are each independently a hydrogen atom, a halogen atom, or an alkyl group,
in the formula (4), at least one selected from R¹³ to R¹⁹ is a group represented by the following formula (5), and the rest are each independently a hydrogen atom, a halogen atom, or an alkyl group, 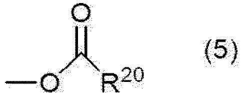
in the formula (5), R²⁰ is a structure of I, II, or III shown below,
I: a linear aliphatic hydrocarbon group having 12 to 22 carbon atoms, a branched aliphatic hydrocarbon group having 12 to 37 carbon atoms, a liposoluble vitamin residue, or a sterol derivative residue,
II: a group represented by the following formula (6) 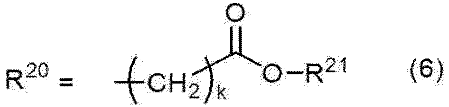 in the formula (6), k is an integer of 2 to 10 indicating the number of repeating units of methylene group, and R²¹ is a linear aliphatic hydrocarbon group having 12 to 22 carbon atoms, a branched aliphatic hydrocarbon group having 12 to 37 carbon atoms, a liposoluble vitamin residue, or a sterol derivative residue,
III: a group containing a benzene ring and a guanidino group, and consisting of 7 or 8 carbon atoms, 3 nitrogen atoms, and a hydrogen atom; 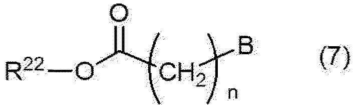
in the formula (7), B is a group represented by the following formula (8), (9), or (10), n is an integer of 1 to 10 indicating the number of repeating units of methylene group, and R²² is methyl or ethyl, 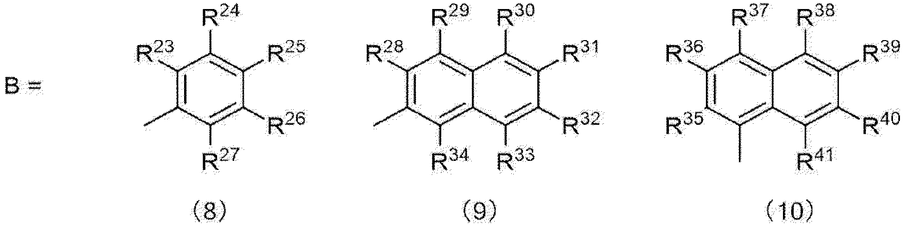
in the formula (8), at least one selected from R²³ to R²⁷ is a hydroxy group, and the rest are each independently a hydrogen atom, a halogen atom, or an alkyl group,
in the formula (9), at least one selected from R²⁸ to R³⁴ is a hydroxy group, and the rest are each independently a hydrogen atom, a halogen atom, or an alkyl group,
in the formula (10), at least one selected from R³⁵ to R⁴¹ is a hydroxy group, and the rest are each independently a hydrogen atom, a halogen atom, or an alkyl group; 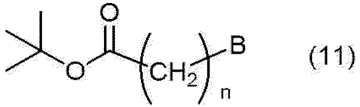
in the formula (11), B is a group represented by the formula (8), (9), or (10), and n is an integer of 1 to 10 indicating the number of repeating units of methylene group; 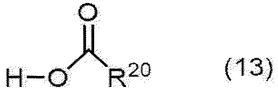
in the formula (13), R²⁰ is a structure of I, II, or III shown in the above; 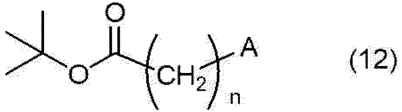
in the formula (12), A is a group represented by the formula (2), (3), or (4), and n is an integer of 1 to 10 indicating the number of repeating units of methylene group.

2. The production method according to claim 1, wherein A in the formulas (1) and (12) is a group represented by the formula (2), and B in the formulas (7) and (11) is a group represented by the formula (8).

3. The production method according to claim 2, wherein, in the formula (2), at least one selected from R¹ to R⁵ is a group represented by the formula (5), and the rest are each independently a hydrogen atom, and in the formula (8), at least one selected from R²³ to R²⁷ is a hydroxy group, and the rest are each independently a hydrogen atom.

4. The production method according to claim 2 or 3, wherein, in the formula (2), R³ is a group represented by the formula (5), and R¹, R², R⁴, and R⁵ are each independently a hydrogen atom, and in the formula (8), R²⁵ is a hydroxy group, and R²³, R²⁴, R²⁶, and R²⁷ are each independently a hydrogen atom.

5. The production method according to any one of claims 1 to 4, wherein R²⁰ in the formulas (5) and (13) is a structure of the I or II.

6. The production method according to any one of claims 1 to 5, wherein n is 1.

7. The production method according to any one of claims 1 to 6, wherein methanesulfonic acid is used in the (step 3).

8. The production method according to any one of claims 1 to 7, wherein the aprotic solvent in the (step 1) is toluene.

9. The production method according to any one of claims 1 to 8, wherein the condensing agent in the (step 2) is selected from the group consisting of 1-(3-dimethylaminopropyl)-3-ethylcarbodiimide hydrochloride (EDC hydrochloride), 1-(3-dimethylaminopropyl)-3-ethylcarbodiimide (EDC), N,N'-dicyclohexylcarbodiimide (DCC), and N,N'-diisopropylcarbodiimide (DIC).

10. The production method according to any one of claims 1 to 9, wherein the organic solvent in the (step 3) is acetonitrile, dichloromethane or chloroform, or a mixture thereof.

11. The production method according to any one of claims 1 to 10, wherein, R²² in the formula (7) is methyl.

12. A method for producing a cationic lipid, comprising the (step 1), (step 2) and (step 3) according to any one of claims 1 to 11, and
(step 4) a step of condensing the obtained aryl ester-containing carboxylic acid represented by the formula (1) and a compound having a disulfide bond, at least one tertiary nitrogen, and at least one hydroxy group or amino group.

13. The production method according to claim 12, wherein the compound is a compound represented by the formula (14), and
in the (step 4), the aryl ester-containing carboxylic acid and the hydroxy groups of the compound are condensed to obtain a cationic lipid represented by the formula (20): 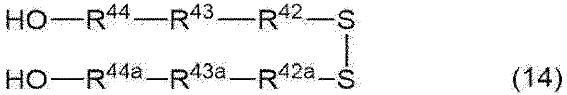
in the formula (14),
R⁴² and R^{42a} are each independently an alkylene group having 1 to 6 carbon atoms,
R⁴³ and R^{43a} are each independently a non-cyclic alkyl tertiary amino group having 1 to 6 carbon atoms and one tertiary amino group, or a cyclic alkylene tertiary amino group having 2 to 5 carbon atoms and 1 to 2 tertiary amino groups, and
R⁴⁴ and R^{44a} are each independently an alkylene group or an oxydialkylene group each having not more than 8 carbon atoms; 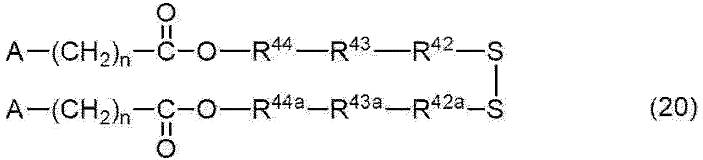
in the formula (20), each symbol is as defined above.
